# EUROPEAN PATENT SPECIFICATION

(11) **EP 1 313 481 B1**
(45) Date of publication and mention of the grant of the patent: **26.09.2007**
(21) Application number: 01954214.1
(22) Date of filing: 08.08.2001
(51) Int. Cl.: A61K 31/5365, A61K 9/20

(54) **PHARMACEUTICAL COMPOSITION COMPRISING CONDENSED INDOLE COMPOUND**
EINE KONDENSIERTE INDOLVERBINDUNG ENTHALTENDES ARZNEIMITTEL
COMPOSITION PHARMACEUTIQUE CONTENANT UN COMPOSE INDOLIQUE CONDENSE

(30) Priority: 08.08.2000 GB 0019524; 02.08.2001 GB 0118919; 03.08.2001 GB 0119022; 07.08.2001 WO PCT/GB01/03544
(43) Date of publication of application: 28.05.2003
(73) Proprietor: SMITHKLINE BEECHAM PLC, Brentford, Middlesex TW8 9GS (GB)
(72) Inventor: BUXTON, Philip, Christopher,GlaxoSmithKline, Harlow C M19 5AW (GB); VAN SCHIE, Dirk, Marinus, J., GlaxoSmithKline, Harlow CM19 5AW (GB); THOMSON, Seona, GlaxoSmithKline, Harlow CM19 5AW (GB)
(74) Representative: Breen, Anthony Paul
(86) International application number: PCT/GB2001/003590
(87) International publication number: WO 2002/011733

(56) References cited:
- EP-A- 0 104 053
- WO-A-93/18036
- P.H. LIST ET AL.: "Hagers Handbuch der Pharmazeutischen Praxis Fourth Edition Vol. 7, Part A" 1971 , SPRINGER-VERLAG , BERLIN (DE) XP002186728 "Granulate" page 312 -page 313
- COOPER ET AL: GASTROENTEROLOGY, vol. 116, no. 4, - 1999 page G2620,
- BHARUCHA ET AL: GASTROENTEROLOGY, vol. 116, no. 4, 1999, page G1470,
- REMINGTON'S PHARMACEUTICAL SCIENCES, 1980, pages 1553-1584,

## Description

This invention relates to a novel composition, for example a tablet or capsule, comprising SB 207266 or a pharmaceutically acceptable salt thereof.

### Introduction

WO 93/18036 (SmithKline Beecham) discloses a large number of condensed indole compounds as 5-HT4 antagonists including, as Example 3 on pages 17-18, N-[(1ⁿbutyl-4-piperidinyl)methyl]-3,4-dihydro-2H-[1,3]oxazino[3,2-a]indole-10-carboxamide (SB 207266) and its preferred hydrochloride salt (SB 207266-A). These compounds are disclosed for use in the treatment or prophylaxis of gastrointestinal, cardiovascular and CNS disorders, in particular irritable bowel syndrome. WO 93/18036 also states in the general description on pp.6-7 in general terms that: "Specific cardiac 5-HT₄ receptor antagonists which prevent atrial fibrillation and other atrial arrhythmias associated with 5-HT would also be expected to reduce the occurrence of stroke". See also US 5,852,014, EP 0 884 319 A2, L.M. Gaster et al, J. Med. Chem., 1995, 38, 4760-4763 and Drugs of the Future, 1997, 22(12), 1325-1332 for the compound SB 207266, which is highly selective for the 5HT₄ receptor over other 5HT receptors. The structure of SB 207266 is as follows:

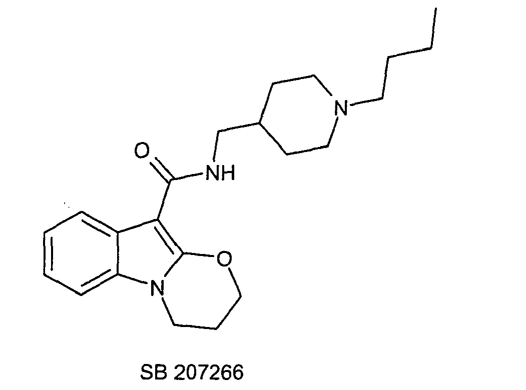

For improved syntheses of SB 207266, see WO 98/07728, WO 98/11067; WO 00/03983; and WO 00/03984.

There are several methods of making the SB 207266 in free base form or as a hydrochloride salt disclosed in the art. Example 3 on page 17-18 of WO 93/1 8036 discloses the production of SB 207266 in free base form in Methods 1 and 2. Method 2 also discloses conversion to the HCl salt and recrystallisation from ethanol/60-80 petrol to give a white solid. L. Gaster, Drugs of the Future, 1997, 22(12), 1325-1332 discloses a similar method involving HCL salt formation by treatment of SB 207266 free base with anhydrous HCL in ethanol. WO 98/07728 discloses three new methods for making the free base on page 6 line 5 to page 7 line 20. WO 98/07728 also discloses two methods of making the HCl salt (SB 207266 A) - Method A on page 7 line 22 to page 8 line 9, and Method B on page 8 line 10 to page 8 line 19. In page 8 lines 10-19 of WO 98/07728, Method B for making the SB 207266 HCl salt is as follows: "N-[(1-Butyl-4-piperidinyl)methyl]-3,4-dihydro-2*H*-[1,3]-oxazino[3,2-*a*]indole-10-carboxamide (SB-207266) (100g, 0.27mol) was dissolved in ethanol (870ml) and the resulting solution filtered to remove particulates. Anhydrous HCl in ethanol (83ml, 3.6M, 0.30mol) was added causing the product to precipitate out of solution. The slurry was heated to redissolve the solid and hexane (550ml) was added. After cooling to room temperature, the mixture was cooled to 0 - 5°C and stirred at that temperature for about two hours. The solid was isolated by filtration and dried *in vacuo* at about 40°C to give the product, N-[(1-butyl-4-piperidinyl)methyl]-3,4-dihydro-2*H*-[1,3]-oxazino[3,2-*a*]indole-10-carboxamide hydrochloride, (102.8g) in 94% yield."

### The Invention

It has now been recognised that there are problems with certain processes for making the SB 207266 HCl salt, which processes are similar or identical to the process disclosed as Method B in page 8 lines 10-19 of WO 98/07728 in that the HC1 salt is dissolved in ethanol, industrial methylated spirits (IMS, e.g. ethanol containing ca. 1% methanol) or similar and crystallised by addition of a C₅-C₁₀ hydrocarbon (e.g. hexane and/or heptane) and/or a solvent containing a C₅-C₁₀ hydrocarbon (e.g. hexane and/or heptane).

The first aspect of the newly recognised problem is that such processes produce the SB 207266 hydrochloride salt in the form of particles of extremely small particle size. For example, the following table shows the particle size data from batches of SB-207266-A made using a process similar to Method B of page 8 of WO 98/07728 but using heptane instead of hexane in the crystallisation step:

| **Batch** | **DV 90 (µm)** | **DV 50 (µm)** | **DV 10 (µm)** |
|---|---|---|---|
| BDC-H-01C | 12.8 | 5.3 | 1.4 |
| BDC-G-02C | 13.8 | 5.7 | 1.5 |
| BDC-G-03C | 16.4 | 6.8 | 1.8 |
| BDC-G-04C | 14.4 | 5.3 | 1.4 |
| BDC-G-05C | 14.6 | 5.8 | 1.5 |
| Average | 14.4 | 5.8 | 1.5 |

DV 90, DV 50 and DV 10 respectively mean that 90%, 50% and 10% by volume of the material is less than the micron size specified.

The second aspect of the newly recognised problem, is the discovery that the SB 207266 HCl salt produced by these processes is very cohesive and has poor flowability / flow characteristics.

The third aspect of the newly recognised problem is that at above certain concentrations in a pharmaceutical formulation, this cohesive drug material causes the composition to be sufficiently poorly-flowing that it cannot easily be tabletted or made into capsules, when the SB-207266 HCl salt is combined with standard methylcellulose, mannitol and Mg stearate excipients. It has been found that a composition for SB 207266, for human oral administration, containing: SB-207266 HCl salt (ca. 5.0 mg), Microcrystalline cellulose (30.0 mg), Mannitol (112.0mg), Mg Stearate (3.0 mg), with total tablet weight = ca. 150 mg , is possible to tablet. However, higher concentrations of the SB-207266 HCl salt are not easily tabletted using this type of formulation.

The fourth aspect of the newly recognised problem is that the small-particle size SB-207266 HCl salt has a low bulk density, densifying on the addition of water. This means that less material can be added to a mixer of fixed volume, leading to a less efficient manufacturing process as large volumes of equipment have to be used for relatively small volumes of drug (smaller throughput in plant).

It has now been discovered that some or all of these problems can be at least partly overcome or mitigated by the forming the SB 207266 HC1 salt into granules which have a particler size larger than than of the original SB 207266 HC1 salt, e.g. by using a wet granulation process. These granules are found to have better flow characteristics for e.g. tabletting purposes. It has also been found that the incorporation of a filler into the granules, especially an insoluble filler such as CaHPO₄ and/or Ca₃(PO₄)₂, can help to form granules with pharmaceutically advantageous properties, e.g. often minimising dissolution of the very soluble SB 207266 HC1 salt in the granulation solvent and so minimising undesirable fusion of granules after removal of the solvent. Some or all of these advantages are also expected to be gained for the free base which is believed also to have usually a small-particle size , e.g. the free base is very slow to filter when crystallised by the addition of hexane to a toluene solution (e.g. as in Method A on page 6 lines 19-23 and Method C on page 7 lines 14-20 of WO 98/07728). Similarly salts other than the HC1 salt are thought to benefit too.

Therefore, **a first aspect of the invention** provides a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable carriers, wherein at least some of the SB 207266 or the salt thereof is in granulated form, and wherein the SB 207266 or salt thereof is present in the composition in at least 4 weight % by weight of the composition,
wherein the granules containing the SB 207266 or the salt thereof also contain a filler (diluent) which is a pharmaceutically acceptable calcium or magnesium salt which is a phosphate, hydrogen phosphate, carbonate or hydrogen carbonate salt, and which is insoluble, practically insoluble, very slightly soluble or slightly soluble in water and/or ethanol, and wherein the weight ratio of the calcium or magnesium salt filler to the drug (the SB 207266 or the salt thereof) in the granules is at least 1:3.

Preferably the composition is a tablet, or the invention can be a capsule containing said composition.

Preferably, the granules including the SB 207266 or salt thereof have a particle size defined by a "D50", or mean particle size e.g. by weight (DM50) or by volume (DV50), of ≥ 100 microns (micrometres) e.g. 100 to 1000 microns , more preferably ≥ 200 microns e.g. 200 to 1000 or 200 to 500 microns, still more preferably ≥ 250 microns e.g. 250 to 500 microns. Preferably, 50% by weight or by volume of the granules including the SB 207266 or salt thereof have a particle size in the specified size range.

Preferably, the granules including the SB 207266 or salt thereof have a particle size defined by a "D10", e.g. by weight (DM10) or by volume (DV10), of ≥ 10 microns (micrometres) e.g. 10 to 1000 microns, more preferably ≥ 50 microns e.g. 50 to 1000 or 50 to 500 microns, still more preferably ≥ 100 microns e.g. 100 to 500 microns.

Compositions of the invention containing granules with the above-mentioned medium to large particle sizes are generally less cohesive, flow better, and are thus less likely to cause the above-mentioned formulation problems.

Preferably, the particles of the SB 207266 or salt thereof (e.g. before forming into granules and/or after granule formation; e.g. within the granules) have a particle size defined by a "D50", or mean particle size e.g. by weight (DM50) or by volume (DV50), of ≤ 80 microns (micrometres), more preferably ≤ 50 microns, still more preferably ≤ 20 microns, even more preferably ≤ 10 microns, most preferably ≤ 8 microns. Preferably, 50% by weight or by volume of the particles of the SB 207266 or salt thereof (e.g. before forming into granules and/or after granule formation; e.g. within the granules) have a particle size in the specified size range.

Preferably, the particles of the SB 207266 or salt thereof (e.g. before forming into granules and/or after granule formation; e.g. within the granules) have a particle size defined by a "D10", e.g. by weight (DM10) or by volume (DV10), of ≤ 20 microns (micrometres), more preferably ≤ 10 microns, still more preferably ≤ 5 microns, even more preferably ≤ 2.5 microns, most preferably ≤ 2 microns. Preferably, 10% by weight or by volume of the particles of the SB 207266 or salt thereof (e.g. before forming into granules and/or after granule formation; e.g. within the granules) have a particle size in the specified size range.

Preferably, the particles of the SB 207266 or salt thereof (e.g. before forming into granules and/or after granule formation; e.g. within the granules) have a particle size defined by a "D90", e.g. DV90 or DM90, of ≤ 100 microns (micrometres), more preferably ≤ 50 microns, still more preferably ≤ 20 microns. Preferably, 90% by weight or by volume of the particles of SB 207266 or salt thereof (e.g. before forming into granules and/or after granule formation; e.g. within the granules) have a particle size in the specified size range.

As discussed above, SB 207266 or salts with such small particle sizes are the ones most likely to give the problems above-mentioned, and are most likely to benefit from the present invention.

In general, particle sizes (D50, D10, D90, et al.) can be measured by sieving with one or more sieves (e.g. for granules before further processing into tablets, and/or for measuring the powder inside capsules). For a tablet, particle sizes can be measured directly (e.g. optically e.g. by microscope, or otherwise) in for example a section through the tablet - diameters of specific particles can be measured which enables an estimation of the particle size distibution by volume and thence by weight.

The SB 207266 or salt thereof is present in the composition in at least 4 weight %, or more preferably at least 6 weight % or at least 8 weight %, by weight of the composition. Preferably, the SB 207266 or salt thereof is present in the composition in up to 95 weight %, more preferably up to 70 weight %, most preferably up to 50 weight %. For example, about 10-100 mg (e.g. 10, 20, 25, 40, 50, 75, 80 and 100mg) of SB 207266 or salt thereof (measured either as the free base or as the actual weight including counterions) for every 250mg of weight of composition (e.g. for every 250 mg coated or uncoated tablet weight) is ideal.

Preferably, the N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof comprises (e.g. is) the hydrochloride salt of SB 207266 (SB 207266-A).

The granules containing the SB 207266 or salt thereof also contain a filler (diluent). Mixing the filler with the SB 207266 or salt thereof before granulation often aids formation of granules. Granulating pure SB 207266 or a salt is difficult.

Preferably, the filler (diluent) is abrasive. This helps to aleviate the cohesiveness of the SB 207266 or salt.

Preferably, the filler (diluent) is insoluble, practically insoluble, very slightly soluble or slightly soluble (more preferably insoluble or practically insoluble) in a/the granulating solvent, e.g. water and/or ethanol. The terms "practically insoluble", "very slightly soluble" and/or "slightly soluble" can be as defined in the British Pharmacopoeia, the European Pharmacopoeia and/or the US Pharmacopoeia. "Practically insoluble" according to the British Pharmacopoeia 1999 (page 1 1) means that at least 10 litres of the solvent is required to dissolve 1 gram of the filler (e.g. at ambient temperature, e.g. 20 or preferably 25 °C). "Very slightly soluble" according to the British Pharmacopoeia means that at least 1 litre and up to 10 litres of the solvent is required to dissolve 1 gram of the filler (e.g. at 25 °C). "Slightly soluble" according to the British Pharmacopoeia means that at least 100 ml and up to 1 litre of the solvent is required to dissolve 1 gram of the filler (e.g. at 25 °C).

The practically insoluble, very slightly soluble or slightly soluble (preferably insoluble) filler, for (wet) granulation, often minimises/reduces undesirable fusion of granules after removal of the granulation solvent and/or improves the quality of the granules.

Preferably, the filler comprises any pharmaceutically acceptable calcium or magnesium salt which is insoluble, practically insoluble, very slightly soluble or slightly soluble (preferably insoluble) in the granulating solvent e.g. water and/or ethanol. The salt can for example be a phosphate, hydrogen phosphate, carbonate or hydrogen carbonate salt. Such insoluble-to-slightly soluble salts include calcium phosphate, dibasic calcium phosphate, calcium carbonate, magnesium carbonate, magnesium phosphate, etc.

Preferably, the filler comprises dibasic calcium phosphate (i.e. dicalcium phosphate, CaHPO₄), more preferably dibasic calcium phosphate hydrate e.g. dihydrate (i.e.CaHPO₄.2H₂O). Anhydrous dibasic calcium phosphate can also be used. CaHPO₄ , e.g. hydrated or anhydrous, is abrasive and helps to aleviate the cohesiveness of the SB 207266 or salt; and it is insoluble in water which helps the granulation process as described above. Alternatively or additionally, the filler can comprise calcium phosphate, i.e. tribasic calcium phosphate, Ca₃(PO₄)₂.

The filler is preferably present in up to 95% by weight of the granules and/or up to 70% by weight of the composition. Preferably, the filler is present in ≥ 15 wt% or ≥ 20 wt% or ≥ 30 wt% of the composition. The weight ratio of filler to drug in the composition or granules is at least 1:3, preferably at least 1:2.5 or at least 1:2 or at least 2:3.

Preferably, the composition includes an excipient which acts as a compression and/or granulation aid, such as microcrystalline cellulose (MCC), preferably present in at least 15wt%, more preferably 15-30 wt% (e.g. about 20 wt%) of the composition. MCC acts to help plastic deformation when tabletting. The compression and/or granulation aid can be present inside or outside the granules.

Preferably, the composition includes a binder such as hydroxypropylmethylcellulose (HPMC) (e.g. low viscosity HPMC such as Pharmacoat 603). The binder in preferably present in the granules. Other possible binders can include HPC, HEC, HMC, methyl cellulose, ethyl cellulose, etc. The binder can preferably be present in about 2.5 to about 10 weight % (e.g. about 5 wt%) of the composition

Preferably, the composition includes a disintegrant (e.g. tablet disintegrant) such as sodium starch glycollate. The disintegrant can be preferably present in about 2.5 to about 10 weight % (e.g. about 5 wt%) of the composition.

Preferably, the composition includes a lubricant such as magnesium stearate. The lubricant can be preferably present in about about 0.2 to about 2 weight % (e.g. about 1 wt%) of the composition.

**A second aspect of the invention** provides a method of making a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable carriers, and wherein the SB 207266 or the salt thereof is present in the composition in at least 4 weight % by weight of the composition,
the method comprising forming at least some of the SB 207266 or the salt thereof into granules,
wherein the granules containing the SB 207266 or the salt thereof also contain a filler (diluent) which is a pharmaceutically acceptable calcium or magnesium salt which is a phosphate, hydrogen phosphate, carbonate or hydrogen carbonate salt, and which is insoluble, practically insoluble, very slightly soluble or slightly soluble in water and/or ethanol,
and wherein the weight ratio of the calcium or magnesium salt filler to the drug (the SB 207266 or the salt thereof) in the granules is at least 1:3,
and wherein the method comprises mixing some or all of the SB 207266 or the salt thereof with the calcium or magnesium salt filler (diluent) before granulation.

Preferably, the method also comprises mixing some or all of the SB 207266 or salt thereof with a filler and/or a binder before granulation. The filler and/or binder can be as defined above.

Preferably, the granules are formed in the presence of a granulating solvent (i.e. using a "wet granulation" process), e.g. comprising or being water and/or ethanol, preferably water. The solvent can be added after mixing of the SB 207266 or salt with the filler and/or binder. Preferably, just sufficient solvent to enable granulation is used.

Preferably, the solvent is removed after formation of the granules, e.g. by drying.

Preferably, the composition/granules are then optionally mixed with other excipient(s) and compressed into tablets.

SB 207266 or the salt thereof may conveniently be administered by any of the routes conventionally used for drug administration, for instance, parenterally, orally, topically or by inhalation.

Procedures for making the composition and/or tablet and/or capsule may involve mixing, granulating and compressing or dissolving the ingredients as appropriate to the desired preparation.

The excipient(s)/carriers used in the composition should be "pharmaceutically acceptable" in the sense of being compatible with the other ingredients of the formulation and not deleterious to the recipient thereof.

The pharmaceutically acceptable carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier or diluent may include time delay material well known to the art, such as glyceryl monostearate or glyceryl distearate alone or with a wax.

A wide variety of pharmaceutical forms can be employed. Thus, if a solid carrier is used, the preparation can be tableted, placed in a hard gelatin capsule in powder or pellet form or in the form of a troche or lozenge. The amount of solid carrier will vary widely but preferably will be from about 25mg to about 1g. When a liquid carrier is used, the preparation will be in the form of a syrup, emulsion, soft gelatin capsule, sterile injectable liquid such as an ampoule or nonaqueous liquid suspension.

A particularly preferred oral composition for SB 207266, for human oral administration, is as follows:

| | |
|---|---|
| SB-207266 | 5.0 mg |
| Microcrystalline cellulose | 50.0 mg |
| HPMC | 12.5 mg |
| Sodium Starch glycollate | 12.5 mg |
| Dicalcium phosphate | 167.5 mg |
| Mg stearate | 2.5 mg |
| | |
| Tablet weight | 250 mg |

### HPMC = hydroxypropylmethylcellulose

The dose in the above composition can readily be increased to 20 mg. This is the result of a granulation process.

This and other suitable oral compositions for SB 207266 are described in the Examples hereinbelow.

All publications, including but not limited to patents and patent appli cations, cited in this specification are herein incorporated by reference as if each individual publication were specifically and individually indicated to be incorporated by reference herein as though fully set forth.

The invention will now be described by reference to the following Examples which are merely illustrative and are not to be construed as a limitation of the scope of the present invention.

### EXAMPLES

SB 207266 - N-[(1-ⁿbutyl-4-piperidinyl)methyl]-3,4-dihydro-2H-[1,3]oxazino[3,2-a]indole-10-carboxamide - is made using the synthetic methods decribed in the introduction, i.e. as described in one or more of WO 98/07728, WO 98/11067; WO 00/03983; and/or WO 00/03984. For the SB 207266 hydrochloride salt, see in particular Method B in page 8 lines 10-19 of WO 98/07728 and minor variations thereof as described above.

### EXAMPLES 1, 2, 3, 3A, 4 and 5 - SB 207266 Pharmaceutical compositions

### Comparative Example 1

An oral composition for SB 207266, for human oral administration, is as follows:

| | |
|---|---|
| SB-207266 | 5.0 mg |
| Microcrystalline cellulose | 30.0 mg |
| Mannitol | 112.0mg |
| Mg Stearate | 3.0 mg |
| | |
| Tablet weight | 150 mg |

This composition is not in accordance with the present invention.

### Example 2

An oral composition for SB 207266, for human oral administration, according to the present invention, is as follows:

| | |
|---|---|
| SB-207266 | 5.0 mg |
| Microcrystalline cellulose | 50.0 mg |
| HPMC (hydroxypropylmethylcellulose) | 12.5 mg |
| Sodium Starch glycollate | 12.5 mg |
| Dicalcium phosphate | 167.5 mg |
| Mg stearate | 2.5 mg |
| | |
| Tablet weight | 250 mg |

The dose in this composition can readily be increased to 20 mg. This composition is the result of a granulation process.

### Example 3

The tablet of Example 2 can be varied by increasing the dose of SB 207266 from 5 mg to up to 20, 60, 75, 80 or 100 mg (measured as the free base), and by decreasing the amount of dicalcium phosphate accordingly while keeping the 250 mg tablet weight constant.
The composition can use SB 207266 as the free base or as the hydrochloride salt.

### Example 3A

The compositions of Examples 2 and 3 can use either SB 207266 as the free base or as the hydrochloride salt.

### Example 4 - SB-207266-A Tablets with 10, 25, and 40mg strength (measured as pure free base)

Tablets containing the hydrochloride salt of SB 207266 (SB 207266-A) in amounts of 10, 25 or 40 mg (measured as the free base) were made according to the composition in the table below.

| *Example 4 composition* | | | | |
|---|---|---|---|---|
| *Ingredient* | *Function* | *Quantity (mg*/*tablet)* | | |
| | | 10 mg tablet strength | 25 mg tablet strength | 40 mg tablet strength |
| *Active Ingredient* SB-207266-A | API | 11.0* | 27.5* | 44.0* |
| | | | | |
| *Other Ingredients* | | | | |
| Microcrystalline Cellulose (e.g. Ph. Eur. or NF) | Compression & granulation aid | 50.0 | 50.0 | 50.0 |
| Hydroxypropylmethyl cellulose (e.g. USP) (e.g. Pharmacoat 603) | Binder | 12.5 | 12.5 | 12.5 |
| Sodium starch glycollate (e.g. NF or Ph Eur) | Disintegrant | 12.5 | 12.5 | 12.5 |
| Calcium hydrogen phosphate dihydrate (Dibasic Calcium Phosphate dihydrate) (e.g. Ph. Eur. or USP) | Major diluent | 161.5 | 145.0 | 128.5 |
| Magnesium Stearate (e.g. Ph. Eur. or NF) | Lubricant | 2.5 | 2.5 | 2.5 |
| Purified Water ** (e.g. Ph. Eur. or USP) | Granulating solvent | ** | ** | ** |
| | | | | |
| Opadry White YS-1-7003 | Film Coat | 6.25 | 6.25 | 6.25 |
| Purified Water ** | | ** | ** | ** |
| | | | | |
| Total Tablet Weight | | 256.25 | 256.25 | 256.25 |

| | | | | |
|---|---|---|---|---|
| * Equivalent to 10, 25, 40mg respectively of pure free base ** Removed during processing | | | | |

The SB-207266-A tablets of Example 4 are packed into high density polyethylene (HDPE) bottles with plastic, child-resistant, induction seal caps.

The formulation used a wet granulation process using an insoluble major excipient, Dibasic calcium Phosphate dihydrate (or Dicalcium phosphate). Dibasic calcium Phosphate dihydrate is the major diluent together with microcrystalline cellulose which is added to disperse the granulating solvent and to aid in the overall compressibility. The binding agent added is hydroxypropylmethyl cellulose and the granulation is carried out in a conventional mixer granulator. The granule mix is dried, screened and mixed with sodium starch glycollate as a disintegrant and magnesium stearate as a lubricant to form the compression mix. Tablets are produced on a suitable rotary tablet press, and can be either oval or round in shape.

### Example 4 - Detailed Manufacturing Process, In-process Controls, and Assembly Process

SB-207266-A, microcrystalline cellulose, dibasic calcium phosphate dihydrate, and hydroxypropylmethyl cellulose are blended together. Purified water is added to the blended powders while mixing in a high shear mixer-granulator. The granules are dried in a fluid bed drier and are then transferred to a mixer, where they are blended with sodium starch glycollate and magnesium stearate. The lubricated mix is compressed into tablet cores using a rotary tablet press. The tablet cores are film coated using an aqueous dispersion of Opadry White YS-1-7003.

### Procedure:

- 1.0: Granulation.
1.1 Blend the SB-207266, microcrystalline cellulose, hydroxypropylmethyl cellulose and dibasic calcium phosphate dihydrate in a suitable high shear mixer-granulator.
1.2 Add the purified water to effect the granulation.
1.3 Dry the granules in a fluid bed drier.
1.4 Pass the dried granules through a stainless steel screen using a suitable mill.
1.5 Determine the yield of the granules.
- 2.0: Manufacture of Compression Mix.
2.1 Blend the required quantites of sodium starch glycollate and magnesium stearate with the dried granules
2.2 Determine the yield of compression mix.
- 3.0: Tablet Compression.
3.1 Transfer the compression mix to a suitable tablet machine.
3.2 Compress the tablets.
3.3 Determine the yield of the compressed tablets.
- 4.0: Film Coating.
4.1 Transfer the tablet cores to a suitable coating machine.
4.2 Rotate the cores and spray on aqueous dispersion of Opadry.
4.3 Release test samples are taken randomly from the batch and appropriately labelled.
- 5.0: Bottle filling
5.1 HDPE bottles are filled to the appropriate fill count, induction sealed and fitted with a child resistant cap using suitably automated equipment.

### Example 5

In a modification of Example 4, formulations containing 20mg, 50mg, 75 mg, 80 mg and 100 mg SB-207266 (as the hydrochloride salt, but the dose given being measured as the free base) can been used to make tablets. These formulations maintain (a) the total coated tablet weight of 256.25 mg, (b) the total pre-coating tablet weight of 250 mg and (c) the other excipient amounts in the Example 4 compositions, but adjust the amount of Dibasic Calcium Phosphate dihydrate used as the amount of SB 207266 varies. These tablets can be round or oval.

## Claims

1. A pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable carriers, wherein at least some of the SB 207266 or the salt thereof is in granulated form, and wherein the SB 207266 or the salt thereof is present in the composition in at least 4 weight % by weight of the composition,
wherein the granules containing the SB 207266 or the salt thereof also contain a filler (diluent) which is a pharmaceutically acceptable calcium or magnesium salt which is a phosphate, hydrogen phosphate, carbonate or hydrogen carbonate salt, and which is insoluble, practically insoluble, very slightly soluble or slightly soluble in water and/or ethanol,
and wherein the weight ratio of the calcium or magnesium salt filler to the drug (the SB 207266 or the salt thereof) in the granules is at least 1;3,

2. A composition as claimed in claim 1, wherein the granules including the SB 207266 or the salt thereof have a particle size defined by a "D50", by weight (DM50) or by volume (DV50), of ≥ 100 microns (micrometres).

3. A composition as claimed in claim 1, wherein the granules including the SB 207266 or the salt thereof have a particle size defined by a "D50", by weight (DM50) or by volume (DV50), of 100 to 1000 microns.

4. A composition as claimed in any preceding claim, wherein the granules including the SB 207266 or the salt thereof have a particle size defined by a "D10", by weight (DM 10) or by volume (DV10), of ≥ 10 microns (micrometres).

5. A composition as claimed in any of claims 1 to 3, wherein the granules including the SB 207266 or the salt thereof have a particle size defined by a "D10", by weight (DM10) or by volume (DV10), of 10 to 1000 microns.

6. A composition as claimed in any of claims 1 to 3, wherein the granules including the SB 207266 or the salt thereof have a particle size defined by a "D10", by weight (DM10) or by volume (DV10), of 50 to 500 microns.

7. A composition as claimed in any preceding claim, wherein the particles of the SB 207266 or the salt thereof have a particle size defined by a "D50", by weight (DM50) or by volume (DV50), of ≤ 80 microns (micrometres).

8. A composition as claimed in any preceding claim, wherein the particles of the SB 207266 or the salt thereof have a particle size defined by a "D50", by weight (DM50) or by volume (DV50), of ≤ 50 microns (micrometres),

9. A composition as claimed in any preceding claim, wherein the particles of the SB 207266 or the salt thereof have a particle size defined by a "D10", by weight (DM10) or by volume (DV10), of ≤ 10 microns (micrometres).

10. A composition as claimed in any preceding claim, wherein the particles of the SB 207266 or the salt thereof have a particle size defined by a "D90", either as DV90 (by volume) or as DM90, of ≤ 100 microns (micrometres).

11. A composition as claimed in any preceding claim, wherein the particles of the SB 207266 or the salt thereof within the granules have a particle size defined in any of claims 7 to 10.

12. A composition as claimed in any preceding claim, wherein the SB 207266 or the salt thereof comprises the hydrochloride salt of SB 207266.

13. A composition as claimed in any preceding claim, wherein the SB 207266 or the salt thereof is the hydrochloride salt of SB 207266.

14. A composition as claimed in claim 12 or 13, wherein the hydrochloride salt of SB 207266 is in a form capable of being made by a process in which the hydrochloride salt is dissolved in ethanol or industrial methylated spirits and crystallised by addition of a C₅-C₁₀ hydrocarbon and/or a solvent containing a C₅-C₁₀ hydrocarbon.

15. A composition as claimed in claim 12 or 13, wherein the hydrochloride salt of SB 207266 has been made by a process in which the hydrochloride salt is dissolved in ethanol or industrial methylated spirits and crystallised by addition of a C₆-C₁₀ hydrocarbon and/or a solvent containing a C₅-C₁₀ hydrocarbon.

16. A composition as claimed in claim 14 or 15, wherein the C₅-C₁₀ hydrocarbon and/or the solvent containing the C₅-C₁₀ hydrocarbon is hexane and/or heptane and/or a solvent containing hexane and/or heptane.

17. A composition as claimed in any preceding claim, wherein the SB 207266 or the salt thereof is in granulated form.

18. A composition as claimed in any preceding claim, wherein the SB 207266 or the salt thereof is present in the composition in at least 6 weight % by weight of the composition.

19. A composition as claimed in any preceding claim, wherein the SB 207266 or the salt thereof is present in the composition in at least 8 weight % by weight of the composition.

20. A composition as claimed in any preceding claim, wherein the SB 207266 or the salt thereof is present in the composition in up to 95 weight % by weight of the composition.

21. A composition as claimed in any preceding claim, wherein the SB 207266 or the salt thereof is present in the composition in up to 70 weight % by weight of the composition.

22. A composition as claimed in any preceding claim, wherein the SB 207266 or the salt thereof is present in the composition in up to 50 weight % by weight of the composition.

23. A composition as claimed in any preceding claim, wherein the calcium or magnesium salt filler (diluent) is abrasive,

24. A composition as claimed in any preceding claim, wherein the calcium or magnesium salt filler (diluent) is insoluble or practically insoluble in water and/or ethanol.

25. A composition as claimed in any of claims 1 to 24, wherein the calcium or magnesium salt filler is insoluble, practically insoluble, very slightly soluble or slightly soluble in water.

26. A composition as claimed in claim 25, wherein the calcium or magnesium salt filler is insoluble or practically insoluble in water.

27. A composition as claimed in any preceding claim, wherein the insoluble-to-slightly soluble pharmaceutically acceptable calcium or magnesium salt filler includes tribasic calcium phosphate (calcium phosphate, Ca₃(PO₄)₂), dibasic calcium phosphate (CaHPO₄), calcium carbonate, magnesium carbonate or magnesium phosphate.

28. A composition as claimed in claim 27, wherein the calcium or magnesium salt filler comprises dibasic calcium phosphate (CaHPO₄) and/or tribasic calcium phosphate (calcium phosphate, Ca₃(PO₄)₂).

29. A composition as claimed in claim 28, wherein the calcium or magnesium salt filler comprises dibasic calcium phosphate (CaHPO₄).

30. A composition as claimed in claim 28, wherein the calcium or magnesium salt filler is dibasic calcium phosphate (CaHPO₄).

31. A composition as claimed in claim 29 or 30, wherein the calcium or magnesium salt filler comprises dibasic calcium phosphate dihydrate (CaHPO₄.2H₂O).

32. A composition as claimed in any of claims 1 to 31, wherein the calcium or magnesium salt filler is present in up to 95% by weight of the granules.

33. A composition as claimed in any of claims 1 to 32, wherein the calcium or magnesium salt filler is present in up to 70% by weight of the composition.

34. A composition as claimed in any of claims 1 to 33, wherein the calcium or magnesium salt filler is present in ≥ 15 weight % of the composition,

35. A composition as claimed in any of claims 1 to 33, wherein the calcium or magnesium salt filler is present in ≥ 20 weight % of the composition.

36. A composition as claimed in any of claims 1 to 35, wherein the weight ratio of the calcium or magnesium salt filler to the drug (the SB 207266 or the salt thereof) in the granules is at least 1 : 2.5.

37. A composition as claimed in any of claims 1 to 35, wherein the weight ratio of the calcium or magnesium salt filler to the drug (the SB 207266 or the salt thereof) in the granules is at least 1:2.

38. A composition as claimed in any of claims 1 to 35, wherein the weight ratio of the calcium or magnesium salt filler to the drug (the SB 207266 or the salt thereof) in the granules is at least 2.3.

39. A composition as claimed in any preceding claim, wherein the composition includes an excipient which acts as a compression and/or granulation aid.

40. A composition as claimed in claim 39, wherein the compression and/or granulation aid is microcrystalline cellulose.

41. A composition as claimed in claim 39 or 40, wherein the compression and/or granulation aid is present in at least 15 weight % of the composition.

42. A composition as claimed in claim 41, wherein the compression and/or granulation aid is present in 15-30 weight % of the composition.

43. A composition as claimed in any of claims 39 to 42, wherein the compression and/or granulation aid is present inside the granules.

44. A composition as claimed in any preceding claim including a binder.

45. A composition as claimed in claim 44, wherein the binder is hydroxypropylmethylcellulose (HPMC), hydroxypropylcellulose (HPC), hydroxyethylcellulose (HEC), hydroxymethylcellulose (HMC), methyl cellulose or ethyl cellulose.

46. A composition as claimed in claim 44, wherein the binder is hydroxypropylmethylcellulose (HPMC).

47. A composition as claimed in claim 46, wherein the binder is low viscosity hydroxypropylmethylcellulose (HPMC).

48. A composition as claimed in claim 44, 45, 46 or 47, wherein the binder is present in 2.5 to 10 weight % of the composition.

49. A composition as claimed in any preceding claim including a disintegrant.

50. A composition as claimed in claim 49, wherein the disintegrant is sodium starch glycollate.

51. A composition as claimed in claim 49 or 50, wherein the disintegrant is present in 2.5 to 10 weight % of the composition.

52. A composition as claimed in any preceding claim including a lubricant.

53. A composition as claimed in claim 52, wherein the lubricant is magnesium stearate.

54. A composition as claimed in claim 52 or 53, wherein the lubricant is present in 0.2 to 2 weight % of the composition.

55. A composition as claimed in any preceding claim being a tablet.

56. A composition as claimed in any preceding claim, wherein the granules have been formed in the presence of a granulating solvent (i.e. using a "wet granulation" process).

57. A composition as claimed in claim 56, wherein the granulating solvent comprises or is water and/or ethanol.

58. A composition as claimed in claim 57, wherein the granulating solvent comprises or is water.

59. A capsule containing a composition as claimed in any preceding claim.

60. A method of making a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable carriers, and wherein the SB 207266 or the salt thereof is present in the composition in at least 4 weight % by weight of the composition,
the method comprising forming at least some of the SB 207266 or the salt thereof into granules,
wherein the granules containing the SB 207266 or the salt thereof also contain a filler (diluent) which is a pharmaceutically acceptable calcium or magnesium salt which is a phosphate, hydrogen phosphate, carbonate or hydrogen carbonate salt, and which is insoluble, practically insoluble, very slightly soluble or slightly soluble in water and/or ethanol,
and wherein the weight ratio of the calcium or magnesium salt filler to the drug (the SB 2.Q7266 or the salt thereof) in the granules is at least 1:3,
and wherein the method comprises mixing some or all of the SH 207266 or the salt thereof with the calcium or magnesium salt filler (diluent) before granulation.

61. A method as claimed in claim 60, wherein the granules are formed in the presence of a granulating solvent (i.e. using a "wet granulation" process).

62. A method as claimed in claim 61, wherein the granulating solvent comprises or is water and/or ethanol.

63. A method as claimed in claim 62, wherein the granulating solvent comprises or is water.

64. A method as claimed in claim 62 or 63, wherein the calcium or magnesium salt filler (diluent) is insoluble, practically insoluble, very slightly soluble or slightly soluble in the granulating solvent used in the "wet granulation" process.

65. A method as claimed in claim 61, 62, 63 or 64, wherein the granulating solvent is added after mixing the SB 207266 or the salt thereof with the calcium or magnesium salt filler and optionally with a binder.

66. A method as claimed in claim 61, 62, 63, 64 or 65, wherein just sufficient solvent to enable granulation is used.

67. A method as claimed in any of claims 61 to 66, wherein the solvent is removed after formation of the granules.

68. A method as claimed in claim 67, wherein the solvent is removed by drying after formation of the granules.

69. A method as claimed in claim 67 or 68, wherein the granules are then optionally mixed with other excipient(s) and compressed into tablets.

70. A method as claimed in any of claims 60 to 69, wherein the filler (diluent) is as defined in any of claims 23 to 38.

71. A method as claimed in any of claims 60 to 70, comprising mixing some or all of the SB 207266 or the salt thereof with a binder before granulation.

72. A method as claimed in claim 71, wherein the binder is as defined in any of claims 45 to 48.

73. A method as claimed in any of claims 60 to 72, wherein the composition includes an excipient which acts as a compression and/or granulation aid.

74. A method as claimed in claim 73, wherein the compression and/or granulation aid is present inside the granules.

75. A method as claimed in claim 73 or 74, wherein the compression and/or granulation aid is as defined in any of claims 40 to 42.

76. A method as claimed in any of claims 60 to 75, wherein the composition is as defined in any of claims 2 to 22 and/or any of claims 49 to 59.

77. A pharmaceutical composition as claimed in any of claims 1 to 55 or claim 59 and capable of being made by a method as defined in any of claims 61 to 76,

78. A pharmaceutical composition as claimed in any of claims 1 to 55 or claim 59 which has been made by a method as defined in any of claims 61 to 76.

## Patentansprüche

1. Pharmazeutische Zusammensetzung, die N-[(1-ⁿButyl-4-piperidinyl)methyl]-3,4-dihydro-2H-[1,3]oxazino[3,2-a]indol-10-carboxamid (SB 207266) oder ein pharmazeutisch annehmbares Salz davon in Kombination mit einem oder mehreren pharmazeutisch annehmbaren Trägern umfaßt, worin zumindest ein Teil des SB 207266 oder des Salzes davon in einer granulierten Form ist, und worin das SB 207266 oder das Salz davon in der Zusammensetzung in mindestens 4 Gew.% der Zusammensetzung vorliegt,
worin die Granalien, die das SB 207266 oder das Salz davon enthalten, ebenfalls einen Füllstoff (Verdünnungsmittel) enthalten, das ein pharmazeutisch annehmbares Calcium- oder Magnesiumsalz ist, das ein Phosphat-, Hydrogenphosphat-, Carbonat- oder Hydrogencarbonatsalz ist, und das unlöslich, praktisch unlöslich, sehr schwach löslich oder schwach löslich in Wasser und/oder Ethanol ist,
und worin das Gewichtsverhältnis des Calcium- oder Magnesiumsalz-Füllstoffs zum Wirkstoff (das SB 207266 oder das Salz davon) in den Granalien mindestens 1:3 ist.

2. Zusammensetzung gemäß Anspruch 1, worin die Granalien, die das SB 207266 oder das Salz davon einschließen, eine Teilchengröße von ≥ 100 Mikron (Mikrometer) haben, die durch einen "D50"-Wert nach Gewicht (DM50) oder nach Volumen (DV50) definiert ist.

3. Zusammensetzung gemäß Anspruch 1, worin die Granalien, die das SB 207266 oder das Salz davon einschließen, eine Teilchengröße von 100 bis 1.000 Mikron haben, die durch einen "D50"-Wert nach Gewicht (DM50) oder nach Volumen (DV50) definiert ist.

4. Zusammensetzung gemäß einem der vorangegangenen Ansprüche, worin die Granalien, die das SB 207266 oder das Salz davon einschließen, eine Teilchengröße von ≥ 10 Mikron (Mikrometer) haben, die durch einen "D10"-Wert nach Gewicht (DM10) oder nach Volumen (DV10) definiert ist.

5. Zusammensetzung gemäß einem der Ansprüche 1 bis 3, worin die Granalien, die das SB 207266 oder das Salz davon einschließen, eine Teilchengröße von 10 bis 1.000 Mikron haben, die durch einen "D10"-Wert nach Gewicht (DM10) oder nach Volumen (DV10) definiert ist.

6. Zusammensetzung gemäß einem der Ansprüche 1 bis 3, worin die Granalien, die das SB 207266 oder das Salz davon einschließen, eine Teilchengröße von 50 bis 500 Mikron haben, die durch einen "D10"-Wert nach Gewicht (DM10) oder nach Volumen (DV10) definiert ist.

7. Zusammensetzung gemäß einem der vorangegangenen Ansprüche, worin die Teilchen des SB 207266 oder des Salzes davon eine Teilchengröße von ≤ 80 Mikron (Mikrometer) haben, die durch einen "D50"-Wert nach Gewicht (DM50) oder nach Volumen (DV50) definiert ist.

8. Zusammensetzung gemäß einem der vorangegangenen Ansprüche, worin die Teilchen des SB 207266 oder des Salzes davon eine Teilchengröße von ≤ 50 Mikron (Mikrometer) haben, die durch einen "D50"-Wert nach Gewicht (DM50) oder nach Volumen (DV50) definiert ist.

9. Zusammensetzung gemäß einem der vorangegangenen Ansprüche, worin die Teilchen des SB 207266 oder des Salzes davon eine Teilchengröße von ≤ 10 Mikron (Mikrometer) haben, die durch einen "D10"-Wert nach Gewicht (DM10) oder nach Volumen (DV10) definiert ist.

10. Zusammensetzung gemäß einem der vorangegangenen Ansprüche, worin die Teilchen des SB 207266 oder des Salzes davon eine Teilchengröße von ≤ 100 Mikron (Mikrometer) haben, die durch einen "D90"-Wert, entweder als DV90 (nach Volumen) oder als DM90, definiert ist.

11. Zusammensetzung gemäß einem der vorangegangenen Ansprüche, worin die Teilchen des SB 207266 oder des Salzes davon innerhalb der Granalien eine Teilchengröße haben, die in einem der Ansprüche 7 bis 10 definiert ist.

12. Zusammensetzung gemäß einem der vorangegangenen Ansprüche, worin das SB 207266 oder das Salz davon das Hydrochloridsalz des SB 207266 umfaßt.

13. Zusammensetzung gemäß einem der vorangegangenen Ansprüche, worin das SB 207266 oder das Salz davon das Hydrochloridsalz des SB 207266 ist.

14. Zusammensetzung gemäß Anspruch 12 oder 13, worin das Hydrochloridsalz des SB 207266 in einer Form ist, die durch ein Verfahren herstellbar ist, in dem das Hydrochloridsalz in Ethanol oder vergälltem Alkohol gelöst und durch Zugabe eines C₅₋₁₀-Kohlenwasserstoffs und/oder eines Lösungsmittels, das einen C₅₋₁₀-Kohlenwasserstoff enthält, kristallisiert wird.

15. Zusammensetzung gemäß Anspruch 12 oder 13, worin das Hydrochloridsalz des SB 207266 durch ein Verfahren hergestellt wurde, worin das Hydrochloridsalz in Ethanol oder vergälltem Alkohol gelöst und durch Zugabe eines C₅₋₁₀-Kohlenwasserstoffs und/oder eines Lösungsmittels, das einen C₅₋₁₀-Kohlenwasserstoff enthält, kristallisiert wird.

16. Zusammensetzung gemäß Anspruch 14 oder 15, worin der C₅₋₁₀-Kohlenwasserstoff und/oder das Lösungsmittel, das den C₅₋₁₀-Kohlenwasserstoff enthält, Hexan und/oder Heptan und/oder ein Lösungsmittel, das Hexan und/oder Heptan enthält, ist.

17. Zusammensetzung gemäß einem der vorangegangenen Ansprüche, worin das SB 207266 oder das Salz davon in granulierter Form ist.

18. Zusammensetzung gemäß einem der vorangegangenen Ansprüche, worin das SB 207266 oder das Salz davon in der Zusammensetzung in mindestens 6 Gew.% der Zusammensetzung vorliegt.

19. Zusammensetzung gemäß einem der vorangegangenen Ansprüche, worin das SB 207266 oder das Salz davon in der Zusammensetzung in mindestens 8 Gew.% der Zusammensetzung vorliegt.

20. Zusammensetzung gemäß einem der vorangegangenen Ansprüche, worin das SB 207266 oder das Salz davon in der Zusammensetzung in bis zu 95 Gew.% der Zusammensetzung vorliegt.

21. Zusammensetzung gemäß einem der vorangegangenen Ansprüche, worin das SB 207266 oder das Salz davon in der Zusammensetzung in bis zu 70 Gew.% der Zusammensetzung vorliegt.

22. Zusammensetzung gemäß einem der vorangegangenen Ansprüche, worin das SB 207266 oder das Salz davon in der Zusammensetzung in bis zu 50 Gew.% der Zusammensetzung vorliegt.

23. Zusammensetzung gemäß einem der vorangegangenen Ansprüche, worin der Calcium- oder Magnesiumsalz-Füllstoff (Verdünnungsmittel) abschleifend ist.

24. Zusammensetzung gemäß einem der vorangegangenen Ansprüche, worin der Calcium- oder Magnesiumsalz-Füllstoff (Verdünnungsmittel) in Wasser und/oder Ethanol unlöslich oder praktisch unlöslich ist.

25. Zusammensetzung gemäß einem der Ansprüche 1 bis 24, worin der Calcium- oder Magnesiumsalz-Füllstoff in Wasser unlöslich, praktisch unlöslich, sehr schwach löslich oder schwach löslich ist.

26. Zusammensetzung gemäß Anspruch 25, worin der Calcium-oder Magnesiumsalz-Füllstoff in Wasser unlöslich oder praktisch unlöslich ist.

27. Zusammensetzung gemäß einem der vorangegangenen Ansprüche, worin der unlösliche bis schwach lösliche pharmazeutisch annehmbare Calcium- oder Magnesiumsalz-Füllstoff dreibasiges Calciumphosphat (Calciumphosphat, Ca₃(PO₄)₂), zweibasiges Calciumphosphat (CaHPO₄), Calciumcarbonat, Magnesiumcarbonat oder Magnesiumphosphat einschließt.

28. Zusammensetzung gemäß Anspruch 27, worin der Calcium-oder Magnesiumsalz-Füllstoff zweibasiges Calciumphosphat (CaHPO₄) und/oder dreibasiges Calciumphosphat (Calciumphosphat, Ca₃(O₄)₂) umfaßt.

29. Zusammensetzung gemäß Anspruch 28, worin der Calcium-oder Magnesiumsalz-Füllstoff zweibasiges Calciumphosphat (CaHPO₄) umfaßt.

30. Zusammensetzung gemäß Anspruch 28, worin der Calcium-oder Magnesiumsalz-Füllstoff zweibasiges Calciumphosphat (CaHPO₄) ist.

31. Zusammensetzung gemäß Anspruch 29 oder 30, worin der Calcium- oder Magnesiumsalz-Füllstoff zweibasiges Calciumphosphatdihydrat (CaHPO₄·2H₂O) umfaßt.

32. Zusammensetzung gemäß einem der Ansprüche 1 bis 31, worin der Calcium- oder Magnesiumsalz-Füllstoff in bis zu 95 Gew.% der Granalien vorliegt.

33. Zusammensetzung gemäß einem der Ansprüche 1 bis 32, worin der Calcium- oder Magnesiumsalz-Füllstoff in bis zu 70 Gew.% der Zusammensetzung vorliegt.

34. Zusammensetzung gemäß einem der Ansprüche 1 bis 33, worin der Calcium- oder Magnesiumsalz-Füllstoff in ≥ 15 Gew.% der Zusammensetzung vorliegt.

35. Zusammensetzung gemäß einem der Ansprüche 1 bis 33, worin der Calcium- oder Magnesiumsalz-Füllstoff in ≥ 20 Gew.% der Zusammensetzung vorliegt.

36. Zusammensetzung gemäß einem der Ansprüche 1 bis 35, worin das Gewichtsverhältnis von dem Calcium- oder Magnesiumsalz-Füllstoff zum Wirkstoff (das SB 207266 oder das Salz davon) in den Granalien mindestens 1:2,5 ist.

37. Zusammensetzung gemäß einem der Ansprüche 1 bis 35, worin das Gewichtsverhältnis von dem Calcium- oder Magnesiumsalz-Füllstoff zum Wirkstoff (das SB 207266 oder das Salz davon) in den Granalien mindestens 1:2 ist.

38. Zusammensetzung gemäß einem der Ansprüche 1 bis 35, worin das Gewichtsverhältnis von dem Calcium- oder Magnesiumsalz-Füllstoff zum Wirkstoff (das SB 207266 oder das Salz davon) in den Granalien mindestens 2:3 ist.

39. Zusammensetzung gemäß einem der vorangegangenen Ansprüche, worin die Zusammensetzung einen Exzipienten einschließt, der als eine Verpressungs- und/oder Granulierungshilfe wirkt.

40. Zusammensetzung gemäß Anspruch 39, worin die Verpressungs- und/oder Granulierungshilfe mikrokristalline Cellulose ist.

41. Zusammensetzung gemäß Anspruch 39 oder 40, worin die Verpressungs- und/oder Granulierungshilfe in mindestens 15 Gew.% der Zusammensetzung vorliegt.

42. Zusammensetzung gemäß Anspruch 41, worin die Verpressungs- und/oder Granulierungshilfe in 15 bis 30 Gew.% der Zusammensetzung vorliegt.

43. Zusammensetzung gemäß Anspruch 39 bis 42, worin die Verpressungs- und/oder Granulierungshilfe innerhalb der Granalien vorliegt.

44. Zusammensetzung gemäß einem der vorangegangenen Ansprüche, die ein Bindemittel einschließt.

45. Zusammensetzung gemäß Anspruch 44, worin das Bindemittel Hydroxypropylmethylcellulose (HPMC), Hydroxypropylcellulose (HPC), Hydroxyethylcellulose (HEC), Hydroxymethylcellulose (HMC), Methylcellulose oder Ethylcellulose ist.

46. Zusammensetzung gemäß Anspruch 44, worin das Bindemittel Hydroxypropylmethylcellulose (HPMC) ist.

47. Zusammensetzung gemäß Anspruch 46, worin das Bindemittel niederviskose Hydroxypropylmethylcellulose (HPMC) ist.

48. Zusammensetzung gemäß Anspruch 44, 45, 46 oder 47, worin das Bindemittel in 2,5 bis 10 Gew.% der Zusammensetzung vorliegt.

49. Zusammensetzung gemäß einem der vorangegangenen Ansprüche, die ein Sprengmittel einschließt.

50. Zusammensetzung gemäß Anspruch 49, worin das Sprengmittel Natriumstärkeglykolat ist.

51. Zusammensetzung gemäß Anspruch 49 oder 50, worin das Sprengmittel in 2,5 bis 10 Gew.% der Zusammensetzung vorliegt.

52. Zusammensetzung gemäß einem der vorangegangenen Ansprüche, die ein Schmiermittel einschließt.

53. Zusammensetzung gemäß Anspruch 52, worin das Schmiermittel Magnesiumstearat ist.

54. Zusammensetzung gemäß Anspruch 52 oder 53, worin das Schmiermittel in 0,2 bis 2 Gew.% der Zusammensetzung vorliegt.

55. Zusammensetzung gemäß einem der vorangegangenen Ansprüche, wobei die Zusammensetzung eine Tablette ist.

56. Zusammensetzung gemäß einem der vorangegangenen Ansprüche, worin die Granalien in Gegenwart eines Granulierungslösungsmittels gebildet worden sind (d.h. unter Verwendung eines "Naßgranulierungs"-Verfahrens).

57. Zusammensetzung gemäß Anspruch 56, worin das Granulierungslösungsmittel Wasser und/oder Ethanol umfaßt oder ist.

58. Zusammensetzung gemäß Anspruch 57, worin das Granulierungslösungsmittel Wasser umfaßt oder ist.

59. Kapsel, die eine Zusammensetzung gemäß einem der vorangegangenen Ansprüche enthält.

60. Verfahren zur Herstellung einer pharmazeutischen Zusammensetzung, die N-[(1-ⁿButyl-4-piperidinyl)methyl]-3,4-dihydro-2H-[1,3]oxazino[3,2-a]indol-10-carboxamid (SB 207266) oder ein pharmazeutisch annehmbares Salz davon in Kombination mit einem oder mehreren pharmazeutisch annehmbaren Trägern umfaßt, und worin das SB 207266 oder das Salz davon in der Zusammensetzung in mindestens 4 Gew.% der Zusammensetzung vorliegt,
wobei das Verfahren das Formen von mindestens einem Teil des SB 207266 oder des Salzes davon zu Granalien umfaßt,
worin die Granalien, die das SB 207266 oder das Salz davon enthalten, ebenfalls einen Füllstoff (Verdünnungsmittel) enthalten, der ein pharmazeutisch annehmbares Calcium- oder Magnesiumsalz ist, das ein Phosphat-, Hydrogenphosphat-, Carbonat- oder Hydrogencarbonatsalz ist, und das in Wasser und/oder Ethanol unlöslich, praktisch unlöslich, sehr schwach löslich oder schwach löslich ist,
und worin das Gewichtsverhältnis von dem Calcium- oder Magnesiumsalz-Füllstoff zum Wirkstoff (das SB 207266 oder das Salz davon) in den Granalien mindestens 1:3 ist,
und worin das Verfahren das Mischen von einem Teil oder allem des SB 207266 oder des Salzes davon mit dem Calcium- oder Magnesiumsalz-Füllstoff (Verdünnungsmittel) vor der Granulierung umfaßt.

61. Verfahren gemäß Anspruch 60, worin die Granalien in Gegenwart eines Granulierungslösungsmittels gebildet werden (d.h. unter Verwendung eines "Naßgranulierungs"-Verfahrens).

62. Verfahren gemäß Anspruch 61, worin das Granulierungslösungsmittel Wasser und/oder Ethanol umfaßt oder ist.

63. Verfahren gemäß Anspruch 62, worin das Granulierungslösungsmittel Wasser umfaßt oder ist.

64. Verfahren gemäß Anspruch 62 oder 63, worin der Calcium-oder Magnesiumsalz-Füllstoff (Verdünnungsmittel) im Granulierungslösungsmittel, das im "Naßgranulierungs"-Verfahren verwendet wird, unlöslich, praktisch unlöslich, sehr schwach löslich oder schwach löslich ist.

65. Verfahren gemäß Anspruch 61, 62, 63 oder 64, worin das Granulierungslösungsmittel nach dem Mischen des SB 207266 oder des Salzes davon mit dem Calcium- oder Magnesiumsalz-Füllstoff und gegebenenfalls mit einem Bindemittel zugegeben wird.

66. Verfahren gemäß Anspruch 61, 62, 63, 64 oder 65, worin gerade ausreichend Lösungsmittel zum Ermöglichen der Granulierung verwendet wird.

67. Verfahren gemäß einem der Ansprüche 61 bis 66, worin das Lösungsmittel nach der Bildung der Granalien entfernt wird.

68. Verfahren gemäß Anspruch 67, worin das Lösungsmittel nach der Bildung der Granalien durch Trocknen entfernt wird.

69. Verfahren gemäß Anspruch 67 oder 68, worin die Granalien dann gegebenenfalls mit (einem) anderen Exzipienten gemischt und zu Tabletten verpreßt werden.

70. Verfahren gemäß einem der Ansprüche 60 bis 69, worin der Füllstoff (Verdünnungsmittel) einer ist, der wie in einem der Ansprüche 23 bis 38 definiert ist.

71. Verfahren gemäß einem der Ansprüche 60 bis 70, das das Mischen von einem Teil oder allem des SB 207266 oder des Salzes davon mit einem Bindemittel vor der Granulierung umfaßt.

72. Verfahren gemäß Anspruch 71, worin das Bindemittel eines ist, das wie in einem der Ansprüche 45 bis 48 definiert ist.

73. Verfahren gemäß einem der Ansprüche 60 bis 72, worin die Zusammensetzung einen Exzipienten einschließt, der als eine Verpressungs- und/oder Granulierungshilfe wirkt.

74. Verfahren gemäß Anspruch 73, worin die Verpressungs-und/oder Granulierungshilfe innerhalb der Granalien vorliegt.

75. Verfahren gemäß Anspruch 73 oder 74, worin die Verpressungs- und/oder Granulierungshilfe eine ist, die wie in einem der Ansprüche 40 bis 42 definiert ist.

76. Verfahren gemäß einem der Ansprüche 60 bis 75, worin die Zusammensetzung eine ist, die wie in einem der Ansprüche 2 bis 22 und/oder einem der Ansprüche 49 bis 59 definiert ist.

77. Pharmazeutische Zusammensetzung gemäß einem der Ansprüche 1 bis 55 oder Anspruch 59 und die durch ein Verfahren gemäß einem der Ansprüche 61 bis 76 herstellbar ist.

78. Pharmazeutische Zusammensetzung gemäß einem der Ansprüche 1 bis 55 oder Anspruch 59, die durch ein Verfahren wie in einem der Ansprüche 61 bis 76 definiert hergestellt wurde.

## Revendications

1. Composition pharmaceutique comprenant du N-[(1-n-butyl-4-pipéridinyl)méthyl]-3,4-dihydro-2H-[1,3]oxazino[3,2-a]indole-10-carboxamide (SB 207266) ou un sel de celui-ci acceptable du point de vue pharmaceutique en combinaison avec un ou plusieurs supports acceptables du point de vue pharmaceutique, dans laquelle au moins une partie du SB 207266 ou du sel de celui-ci est sous une forme granulée, et dans laquelle le SB 207266 ou le sel de celui-ci est présent dans la composition à raison d'au moins 4% en poids de la composition,
dans laquelle les granules contenant le SB 207266 ou le sel de celui-ci contiennent aussi une charge (diluant) qui est un sel de calcium ou de magnésium acceptable du point de vue pharmaceutique qui est un sel phosphate, hydrogénophosphate, carbonate ou hydrogénocarbonate, et qui est insoluble, pratiquement insoluble, très légèrement soluble ou légèrement soluble dans l'eau et/ou l'éthanol,
et dans laquelle le rapport en poids de la charge de sel de calcium ou de magnésium au médicament (SB 207266 ou le sel de celui-ci) dans les granules est d'au moins 1 :3.

2. Composition selon la revendication 1, dans laquelle les granules contenant le SB 207266 ou le sel de celui-ci ont une dimension particulaire définie par un " D50 " en poids (DM50) ou en volume (DV50) ≥ 100 microns (micromètres).

3. Composition selon la revendication 1, dans laquelle les granules contenant le SB 207266 ou le sel de celui-ci ont une dimension particulaire définie par un " D50 " en poids (DM50) ou en volume (DV50) de 100 à 1000 microns (micromètres).

4. Composition selon l'une quelconque des revendications précédentes, dans laquelle les granules contenant le SB 207266 ou le sel de celui-ci ont une dimension particulaire définie par un " D10 " en poids (DM10) ou en volume (DV10) ≥ 10 microns (micromètres).

5. Composition selon l'une quelconque des revendications 1 à 3, dans laquelle les granules contenant le SB 207266 ou le sel de celui-ci ont une dimension particulaire définie par un " D10 " en poids (DM10) ou en volume (DV10) de 10 à 1000 microns (micromètres).

6. Composition selon l'une quelconque des revendications 1 à 3, dans laquelle les granules contenant le SB 207266 ou le sel de celui-ci ont une dimension particulaire définie par un " D10 " en poids (DM10) ou en volume (DV10) de 50 à 500 microns (micromètres).

7. Composition selon l'une quelconque des revendications précédentes, dans laquelle les particules de SB 207266 ou du sel de celui-ci ont une dimension particulaire définie par un " D50 " en poids (DM50) ou en volume (DV50) ≤ 80 microns (micromètres).

8. Composition selon l'une quelconque des revendications précédentes, dans laquelle les particules de SB 207266 ou du sel de celui-ci ont une dimension particulaire définie par un " D50 " en poids (DM50) ou en volume (DV50) ≤ 50 microns (micromètres).

9. Composition selon l'une quelconque des revendications précédentes, dans laquelle les particules de SB 207266 ou du sel de celui-ci ont une dimension particulaire définie par un " D10 " en poids (DM10) ou en volume (DV10) ≤ 10 microns (micromètres).

10. Composition selon l'une quelconque des revendications précédentes, dans laquelle les particules du SB 207266 ou du sel de celui-ci ont une dimension particulaire définie par un " D90 " soit par DV90 (en volume), soit par DM90 (en poids) ≤ 100 microns (micromètres).

11. Composition selon l'une quelconque des revendications précédentes, dans laquelle les particules de SB 207266 ou du sel de celui-ci à l'intérieur des granules ont une dimension particulaire définie dans l'une quelconque des revendications 7 à 10.

12. Composition selon l'une quelconque des revendications précédentes, dans laquelle le SB 207266 ou le sel de celui-ci comprend le sel chlorhydrate de SB 207266.

13. Composition selon l'une quelconque des revendications précédentes, dans laquelle le SB 207266 ou le sel de celui-ci est le sel chlorhydrate de SB 207266.

14. Composition selon la revendication 12 ou 13, dans laquelle le sel chlorhydrate de SB 207266 est sous une forme susceptible d'être préparée par un procédé dans lequel le sel chlorhydrate est dissous dans de l'éthanol ou de l'alcool dénaturé industriel et cristallisé par addition d'un hydrocarbure en C₅₋₁₀ et/ou d'un solvant contenant un hydrocarbure en C₅₋₁₀.

15. Composition selon la revendication 12 ou 13, dans laquelle le sel chlorhydrate de SB 207266 a été préparé par un procédé dans lequel le sel chlorhydrate est dissous dans de l'éthanol ou de l'alcool dénaturé industriel et cristallisé par addition d'un hydrocarbure en C₅₋₁₀ et/ou d'un solvant contenant un hydrocarbure en C₅₋₁₀.

16. Composition selon la revendication 14 ou 15, dans laquelle l'hydrocarbure en C₅₋₁₀ et/ou le solvant contenant l'hydrocarbure en C₅₋₁₀ est l'hexane et/ou l'heptane et/ou un solvant contenant de l'hexane et/ou de l'heptane.

17. Composition selon l'une quelconque des revendications précédentes, dans laquelle le SB 207266 ou le sel de celui-ci est sous une forme granulée.

18. Composition selon l'une quelconque des revendications précédentes, dans laquelle le SB 207266 ou le sel de celui-ci est présent dans la composition à raison d'au moins 6% en poids par poids de la composition.

19. Composition selon l'une quelconque des revendications précédentes, dans laquelle le SB 207266 ou le sel de celui-ci est présent dans la composition à raison d'au moins 8% en poids par poids de la composition.

20. Composition selon l'une quelconque des revendications précédentes, dans laquelle le SB 207266 ou le sel de celui-ci est présent dans la composition en une quantité allant jusqu'à 95% en poids par poids de la composition.

21. Composition selon l'une quelconque des revendications précédentes, dans laquelle le SB 207266 ou le sel de celui-ci est présent dans la composition en une quantité allant jusqu'à 70% en poids par poids de la composition.

22. Composition selon l'une quelconque des revendications précédentes, dans laquelle le SB 207266 ou le sel de celui-ci est présent dans la composition en une quantité allant jusqu'à 50% en poids par poids de la composition.

23. Composition selon l'une quelconque des revendications précédentes, dans laquelle la charge de sel de calcium ou de magnésium (diluant) est abrasive.

24. Composition selon l'une quelconque des revendications précédentes, dans laquelle la charge de sel de calcium ou de magnésium (diluant) est insoluble ou pratiquement insoluble dans l'eau et/ou l'éthanol.

25. Composition selon l'une quelconque des revendications 1 à 24, dans laquelle la charge de sel de calcium ou de magnésium (diluant) est insoluble, pratiquement insoluble, très légèrement soluble ou légèrement soluble dans l'eau.

26. Composition selon la revendication 25, dans laquelle la charge de sel de calcium ou de magnésium est insoluble ou pratiquement insoluble dans l'eau.

27. Composition selon l'une quelconque des revendications précédentes, dans laquelle la charge de sel de calcium ou de magnésium acceptable du point de vue pharmaceutique insoluble à légèrement soluble comprend du phosphate de calcium tribasique (phosphate de calcium, Ca₃(PO₄)₂), du phosphate de calcium dibasique (CaHPO₄), du carbonate de calcium, du carbonate de magnésium ou du phosphate de magnésium.

28. Composition selon la revendication 27, dans laquelle la charge de sel de calcium ou de magnésium comprend du phosphate de calcium dibasique (CaHPO₄) et/ou du phosphate de calcium tribasique (phosphate de calcium, Ca₃(PO₄)₂).

29. Composition selon la revendication 28, dans laquelle la charge de sel de calcium ou de magnésium comprend du phosphate de calcium dibasique (CaHPO₄).

30. Composition selon la revendication 28, dans laquelle la charge de sel de calcium ou de magnésium est du phosphate de calcium dibasique (CaHPO₄).

31. Composition selon la revendication 29 ou 30, dans laquelle la charge de sel de calcium ou de magnésium comprend du phosphate de calcium dibasique dihydraté (CaHPO₄, 2H₂O).

32. Composition selon l'une quelconque des revendications 1 à 31, dans laquelle la charge de sel de calcium ou de magnésium est présente en une quantité allant jusqu'à 95% en poids des granules.

33. Composition selon l'une quelconque des revendications 1 à 32, dans laquelle la charge de sel de calcium ou de magnésium est présente en une quantité allant jusqu'à 70% en poids de la composition.

34. Composition selon l'une quelconque des revendications 1 à 33, dans laquelle la charge de sel de calcium ou de magnésium est présente en une quantité ≥ 15% en poids de la composition.

35. Composition selon l'une quelconque des revendications 1 à 33, dans laquelle la charge de sel de calcium ou de magnésium est présente en une quantité ≥ 20% en poids de la composition.

36. Composition selon l'une quelconque des revendications 1 à 35, dans laquelle le rapport en poids de la charge de sel de calcium ou de magnésium au médicament (le SB 207266 ou le sel de celui-ci) dans les granules est d'au moins 1 :2,5.

37. Composition selon l'une quelconque des revendications 1 à 35, dans laquelle le rapport en poids de la charge de sel de calcium ou de magnésium au médicament (le SB 207266 ou le sel de celui-ci) dans les granules est d'au moins 1 :2.

38. Composition selon l'une quelconque des revendications 1 à 35, dans laquelle le rapport en poids de la charge de sel de calcium ou de magnésium au médicament (le SB 207266 ou le sel de celui-ci) dans les granules est d'au moins 2 :3.

39. Composition selon l'une quelconque des revendications précédentes, dans laquelle la composition comprend un excipient qui joue le rôle d'un aide à la compression et/ou à la granulation.

40. Composition selon la revendication 39, dans laquelle l'aide à la compression et/ou à la granulation est une cellulose microcristalline.

41. Composition selon la revendication 39 ou 40, dans laquelle l'aide à la compression et/ou à la granulation est présente à raison d'au moins 15% en poids de la composition.

42. Composition selon la revendication 41, dans laquelle l'aide à la compression et/ou à la granulation est présente à raison de 15 à 30% en poids de la composition.

43. Composition selon l'une quelconque des revendications 39 à 42, dans laquelle l'aide à la compression et/ou à la granulation est présente à l'intérieur des granules.

44. Composition selon l'une quelconque des revendications précédentes comprenant un liant.

45. Composition selon la revendication 44, dans laquelle le liant est l'hydroxypropylméthylcellulose (HPMC), l'hydroxypropylcellulose (HPC), l'hydroxyéthylcellulose (HEC), l'hydroxyméthylcellulose (HMC), la méthylcellulose ou l'éthylcellulose.

46. Composition selon la revendication 44, dans laquelle le liant est l'hydroxypropylméthylcellulose (HPMC).

47. Composition selon la revendication 46, dans laquelle le liant est une hydroxypropylméthylcellulose (HPMC) de faible viscosité.

48. Composition selon les revendications 44, 45, 46 ou 47, dans laquelle le liant est présent à raison de 2,5 à 10% en poids de la composition.

49. Composition selon l'une quelconque des revendications précédentes comprenant un agent de désagrégation.

50. Composition selon la revendication 49, dans laquelle l'agent de désagrégation est de l'amidon glycolate de sodium.

51. Composition selon la revendication 49 ou 50, dans laquelle l'agent de désagrégation est présent à raison de 2,5 à 10% en poids de la composition.

52. Composition selon l'une quelconque des revendications précédentes comprenant un lubrifiant.

53. Composition selon la revendication 52, dans laquelle le lubrifiant est le stéarate de magnésium.

54. Composition selon la revendication 52 ou 53, dans laquelle le lubrifiant est présent à raison de 0,2 à 2% en poids de la composition.

55. Composition selon l'une quelconque des revendications précédentes qui est sous la forme d'un comprimé.

56. Composition selon l'une quelconque des revendications précédentes, dans laquelle les granules ont été formés en présence d'un solvant de granulation (c'est-à-dire par utilisation d'un procédé de " granulation humide ").

57. Composition selon la revendication 56, dans laquelle le solvant de granulation comprend ou est de l'eau et/ou de l'éthanol.

58. Composition selon la revendication 57, dans laquelle le solvant de granulation comprend ou est de l'eau.

59. Gélule contenant une composition selon l'une quelconque des revendications précédentes.

60. Procédé de préparation d'une composition pharmaceutique comprenant du N-[(1-n-butyl-4-pipéridinyl)méthyl]-3,4-dihydro-2H-[1,3]oxazino[3,2-a]indole-10-carboxamide (SB 207266) ou un sel de celui-ci acceptable du point de vue pharmaceutique en combinaison avec un ou plusieurs supports acceptables du point de vue pharmaceutique, et où le SB 207266 ou le sel de celui-ci est présent dans la composition à raison d'au moins 4% en poids de la composition,
le procédé comprenant la mise sous la forme de granules d'au moins une partie du SB 207266 ou du sel de celui-ci ;
dans lequel les granules contenant le SB 207266 ou le sel de celui-ci contiennent aussi une charge (diluant) qui est un sel de calcium ou de magnésium acceptable du point de vue pharmaceutique qui est un sel phosphate, hydrogénophosphate, carbonate ou hydrogénocarbonate, et qui est insoluble, pratiquement insoluble, très légèrement soluble ou légèrement soluble dans l'eau et/ou l'éthanol,
et dans lequel le rapport en poids de la charge de sel de calcium ou de magnésium au médicament (SB 207266 ou le sel de celui-ci) dans les granules est d'au moins 1 :3,
et dans lequel le procédé comprend le mélange d'une partie ou de la totalité du SB 207266 ou du sel de celui-ci avec la charge de sel de calcium ou de magnésium (diluant) avant la granulation.

61. Procédé selon la revendication 60, dans lequel les granules sont formés en présence d'un solvant de granulation (c'est-à-dire avec un procédé de " granulation humide ").

62. Procédé selon la revendication 61, dans lequel le solvant de granulation comprend ou est de l'eau et/ou de l'éthanol.

63. Procédé selon la revendication 62, dans lequel le solvant de granulation comprend ou est de l'eau.

64. Procédé selon la revendication 62 ou 63, dans lequel la charge de sel de calcium ou de magnésium (diluant) est insoluble, pratiquement insoluble, très légèrement soluble ou légèrement soluble dans le solvant de granulation utilisé dans le procédé de " granulation humide ".

65. Procédé selon la revendication 61, 62, 63 ou 64, dans lequel le solvant de granulation est ajouté après mélange du SB 207266 ou du sel de celui-ci avec la charge de sel de calcium ou de magnésium et éventuellement avec un liant.

66. Procédé selon la revendication 61, 62, 63, 64 ou 65, dans lequel il est utilisé juste assez de solvant pour permettre la granulation.

67. Procédé selon l'une quelconque des revendications 61 à 66, dans lequel le solvant est éliminé après la formation des granules.

68. Procédé selon la revendication 67, dans lequel le solvant est éliminé par séchage après la formation des granules.

69. Procédé selon la revendication 67 ou 68, dans lequel les granules sont ensuite éventuellement mélangés avec un ou plusieurs autres excipients et compressés en comprimés.

70. Procédé selon l'une quelconque des revendications 60 à 69, dans lequel la charge (diluant) est telle que définie dans l'une quelconque des revendications 23 à 38.

71. Procédé selon l'une quelconque des revendications 60 à 70, comprenant le mélange d'une partie ou de la totalité du SB 207266 ou du sel de celui-ci avec un liant avant la granulation.

72. Procédé selon la revendication 71, dans lequel le liant est tel que défini dans l'une quelconque des revendications 45 à 48.

73. Procédé selon l'une quelconque des revendications 60 à 72, dans lequel la composition comprend un excipient qui joue le rôle d'un aide à la compression et/ou à la granulation.

74. Procédé selon la revendication 73, dans lequel l'aide à la compression et/ou à la granulation est présent à l'intérieur des granules.

75. Procédé selon la revendication 73 ou 74, dans lequel l'aide à la compression et/ou à la granulation est tel que défini dans l'une quelconque des revendications 40 à 42.

76. Procédé selon l'une quelconque des revendications 60 à 75, dans lequel la composition est telle que définie dans l'une quelconque des revendications 2 à 22 et/ou dans l'une quelconque des revendications 49 à 59.

77. Composition pharmaceutique selon l'une quelconque des revendications 1 à 55 ou la revendication 59 et susceptible d'être préparée par un procédé tel que défini dans l'une quelconque des revendications 61 à 76.

78. Composition pharmaceutique selon l'une quelconque des revendications 1 à 55 ou la revendication 59 et qui a été préparée par un procédé tel que défini dans l'une quelconque des revendications 61 à 76.
